(19) Europäisches Patentamt

European Patent Office

Office européen des brevets

(11) **EP 1 634 041 B1**

(12) **EUROPÄISCHE PATENTSCHRIFT**

(45) Veröffentlichungstag und Bekanntmachung des Hinweises auf die Patenterteilung:
**19.07.2006 Patentblatt 2006/29**

(21) Anmeldenummer: 04734470.0

(22) Anmeldetag: **22.05.2004**

(51) Int Cl.:
*G01G 17/00* (2006.01)   *G01G 9/00* (2006.01)
*G01N 22/04* (2006.01)

(86) Internationale Anmeldenummer:
**PCT/EP2004/005529**

(87) Internationale Veröffentlichungsnummer:
**WO 2004/106870 (09.12.2004 Gazette 2004/50)**

(54) **MESSEINRICHTUNG ZUR ZERSTÖRUNGSFREIEN BESTIMMUNG DER EINWAAGE IN KAPSELN**

MEASURING DEVICE FOR NON-DESTRUCTIVELY DETERMINING THE NET WEIGHT IN TABLETS

DISPOSITIF DE MESURE POUR LA DETERMINATION NON DESTRUCTIVE DU POIDS NET DANS DES CAPSULES

(84) Benannte Vertragsstaaten:
**AT BE BG CH CY CZ DE DK EE ES FI FR GB GR HU IE IT LI LU MC NL PL PT RO SE SI SK TR**

(30) Priorität: **03.06.2003 EP 03012566**

(43) Veröffentlichungstag der Anmeldung:
**15.03.2006 Patentblatt 2006/11**

(73) Patentinhaber:
• **Boehringer Ingelheim International GmbH**
  **55216 Ingelheim am Rhein (DE)**
  Benannte Vertragsstaaten:
  **AT BE BG CH CY CZ DK EE ES FI FR GB GR HU IE IT LI LU MC NL PL PT RO SE SI SK TR**
• **Boehringer Ingelheim Pharma GmbH & Co.KG**
  **55216 Ingelheim am Rhein (DE)**
  Benannte Vertragsstaaten:
  **DE**

(72) Erfinder: **MUELLER, Hanns-Walter**
**55127 MAINZ (DE)**

(56) Entgegenhaltungen:
WO-A-97/31244       DE-A- 4 004 119
US-A- 5 515 740     US-A1- 2001 000 946

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

**Beschreibung**

Die Erfindung betrifft ein Verfahren zur zerstörungsfreien Nettoverwiegung von Kapseln mit Hilfe von Mikrowellen.

Hinterarund der Erfindung

In vielen industriellen Herstellprozessen ist die Kenntnis des Nettogewichts verpackter Ware (z.B. Schüttgüter, Lebensmittel usw.) von besonderer Bedeutung. Wenn das Taragewicht gegenüber der Einwaage vernachlässigbar klein ist, so genügt in der Regel eine Bruttoverwiegung der verpackten Ware. Sind die Gebinde jedoch sehr klein, wie z.B. bei Arzneimittelwirkstoffe enthaltenden pharmazeutischen Formulierungen, wie beispielsweise Kapseln, so ist die Relation zwischen Oberfläche und Volumen stark zugunsten der Oberfläche verschoben, und das Taragewicht überschreitet die Einwaage leicht um ein Mehrfaches. In solchen Fällen sind die Schwankungen des Taragewichts nicht mehr zu ignorieren. Die Einwaage muß daher entweder durch direkte NettoVerwiegung des Inhalts bestimmt werden oder durch Verwiegung des Meßobjektes vor und nach der Verpackung (Tara- und Bruttoverwiegung).

Naturgemäß wird eine Nettoverwiegung stets auf der Abfüll- oder Verpackungsmaschine erfolgen, wo die eigentliche Dosierung stattfindet. Gleiches gilt aber auch für die Brutto/Tara-Verwiegung, wenn mit hohen Produktionsraten gearbeitet wird, denn andernfalls wäre die richtige Zuordnung zwischen Tara- und Bruttomeßwerten der einzelnen Meßstücke nur schwer oder nur mit hohem Aufwand zu gewährleisten.

Werden bei gleichzeitig geringen Einwaagen im mg-Bereich hohe Produktionsraten (z.B. 100000 Stück/Stunde und mehr) gefordert, so ergeben sich extreme Präzisions- und Schnelligkeitsanforderungen an die Meßapparatur. Im Stand der Technik sind beispielsweise Online-Brutto-Tara-Verwiegesysteme auf Kapselfüllmaschinen bekannt, bei denen ein kapazitives Meßsystem zum Einsatz gelangt. Dort wird das Meßgut vor und nach der Befüllung durch einen Kondensator geführt, aus dessen Kapazitätsveränderung man auf die jeweils eingebrachte Masse zurückrechnet. Solche Verfahren sind jedoch erfahrungsgemäß nur für Einwaagen größer als 50 mg spezifiziert. Moderne gravimetrische Wiegezellen erfüllen deutlich höhere Genauigkeitsspezifikationen. Sie arbeiten jedoch mit einer nur geringen Geschwindigkeit von einigen wenigen Kapseln pro Sekunde. Bei entsprechender Parallelschaltung solcher Wiegezellen sind zwar grundsätzlich höhere Durchsatzgeschwindigkeiten erreichbar, allerdings unterliegen gravimetrische Wiegezellen der Beschränkung, sehr empfindlich auf Vibrationen zu reagieren und im getakteten statt im kontinuierlichen Modus betrieben werden zu müssen. Dadurch sind gravimetrische Wiegezellen auf kontinuierlich arbeitenden Kapselfüllmaschinen nur sehr bedingt einsetzbar.

Es resultiert also ein Bedarf nach einer Verwiegemethode, die - von der Abfüllmaschine losgelöst als Standalone-Lösung fungierend - den Inhalt fertig befüllter und verschlossener Kapsel zu bestimmen in der Lage ist.

Beschreibung der Erfindung

Die vorstehend genannte Aufgabe wird durch das erfindungsgemäße Verfahren gelöst. Die Erfindung betrifft ein Verfahren zur zerstörungsfreien Nettoverwiegung von Kapseln mit Hilfe von Mikrowellen, bei dem in einem ersten Verfahrensschritt durch einen Prozessor gesteuert in einem Mikrowellen-Generator eine elektromagnetische Strahlung variabler Frequenz erzeugt und einem als Resonator ausgebildeten Probenapplikator zugeführt wird, bei dem das aus dem Applikator austretende Mikrowellensignal einer Detektor-Diode zugeführt wird, aus deren Signalen über einen Analog/Digital-Wandler vom Rechner als primäre Meßgrößen b(0) und f(0) ermittelt werden, wobei b(0) die Halbwertsbreite bei der Resonanzfrequenz f(0) des mit einer Messprobe in Wirkverbindung stehenden Applikators ist, dadurch gekennzeichnet, dass mit dem Resonator eine Materialprobe so in Verbindung gebracht wird, dass das elektrische Feld des Resonators beim Übergang in das Material der Probe allgemein parallel zur Oberfläche verläuft und dass aus den primären Messgrössen b(0) und f(0) die Messsignale

$$F = f(L) - f(0) \text{ bzw. } B = b(0) - b(L)$$

erhalten werden, wobei F und B für die Mikrowellenmesssignale der Verstimmung und Verbreiterung der Resonanz stehen, mit $f(L)$ und $b(L)$ gleich konstante materialabhängige Bezugsgrößen,
und bei dem in einem zweiten Schritt eine gravimetrische Bruttoverwiegung zur Bestimmung des Bruttowiegesignals

$$C = m_T + m_N,$$

in dem die Grösse $m_T$ für die Masse des leeren Gebindes und die Grösse $m_N$ für die zu bestimmende Nettomasse steht, durchgeführt wird, und abschließend aus den erhaltenen Messsignalen *F, B und C* die zu bestimmende Nettomasse $m_N$ gemäß nachfolgender Relation

$$m_N = \alpha F + \beta B + \gamma C + \delta$$

rechnerisch bestimmt wird, wobei die Koeffizienten $\alpha$, $\beta$, $\gamma$ und $\delta$ zuvor über Kalibrierung des Messsystems durch lineare Regression aus einer Meßreihe mit Referenzproben bekannter Einwaage bestimmt wurden.

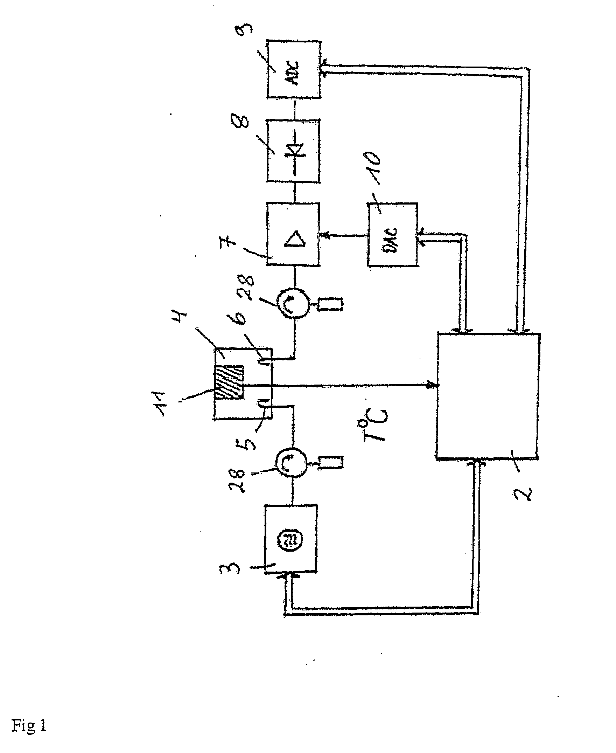

Zur Bestimmung der Meßsignale F und B können Vorrichtungen, wie sie beispielsweise in der DE 4004119 offenbart werden, zum Einsatz gelangen. Figur 1 offenbart den schematischen Aufbau einer solchen Vorrichtung.

Diese Vorrichtung ist, wie in Figur 1 dargestellt, gekennzeichnet durch einen von einem Prozessor (2) digital abstimmbaren Mikrowellengenerator (3) mit variabler Frequenz, der mit einer Ankopplungssonde (5) verbunden ist, die in einem Applikator (4) zur Messung der Meßsignale F und B einer Probe (11) angeordnet ist, der eine weitere Ankopplungssonde (6) aufweist, die über einen Mikrowellen-Verstärker bzw. - Abschwächer (7) mit einer Detektor-Diode (8) verbunden ist, deren Signalausgang mit dem Prozessor (2) verbunden ist. Die Detektor-Diode (8) kann, wie in Figur 1 dargestellt über einen Analog/Digital-Wandler (9) mit dem Prozessor (2) verbunden sein. Der Abschwächer (7) kann, wie in Figur 1 dargestellt über einen Digital/Analog-Wandler (10) mit dem Prozessor (2) verbunden sein. Zwischen dem Mikrowellengeneraqtor (3) und der Ankopplungssonde (5) sowie der Ankopplungssonde (6) und dem Mikrowellen-Verstärker bzw. -Abschwächer (7) ist jeweils zur Entkollpung ein Zirkulator (28) angeordnet.

Zur Bestimmung des Bruttowiegesignals *C* können gravimetrische Wiegezellen zum Einsatz gelangen, die das Gewicht mit einer Standardabweichung von +/- 0.1 mg bestimmen.

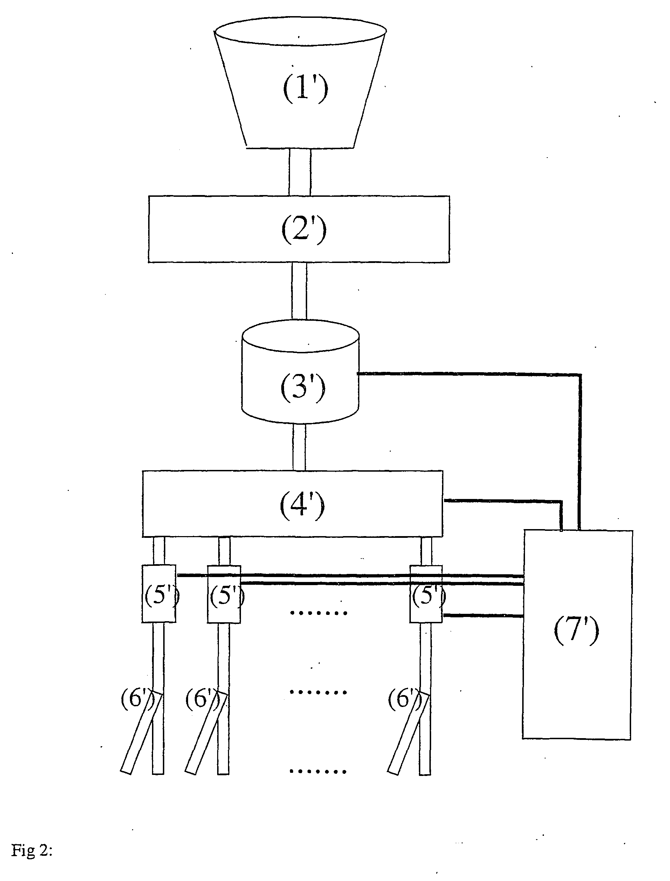

Figur 2 offenbart den schematischen Aufbau der Ankopplung des Mkrowellenverfahrens an die Bruttoverwiegung. Mit Hilfe einer Vereinzelungseinheit (2') werden die befüllten und verschlossenen Kapseln aus einem Trichter (1') seriell und/oder einzeln durch den Mikrowellenresonator (3') geführt. Eine sich daran anschließende Verteilereinheit (4') wie sie in heutigen Kapselwiegemaschinen eingesetzt wird, parallelisiert den Transport und verteilt die Kapseln auf mehrere Spuren, in denen sich die gravimetrischen Wiegezellen (5') befinden. Eine computergestützte Datenerfassungseinheit (7'), die mit den Einheiten (3'), (4') und (5') verbunden ist, sorgt für die richtige Zuordnung der Meßwerte zu den einzelnen Kapseln und führt die Auswertung durch. Kapseln mit fehlgewichtiger Einwaage werden durch eine Weiche (6') ausgeschieden.

Unter Mikrowellenresonator (3') wird dabei eine Vorrichtung gemäß Figur 1 verstanden.

Dieses Verfahren ist geeignet für Einwaagen bis hinunter in den mg-Bereich: Es arbeitet mit hoher Geschwindigkeit und ist selbst dann einsetzbar, wenn das Gewicht der Verpackung bzw. der leeren Kapsel die Einwaage des Kapselinhalts weit überschreitet.

Der besondere Nutzen des Verfahrens besteht darin, dass die erforderliche Messung mit hoher Geschwindigkeit und Präzision durchführen zu können, so daß auch noch Einwaagen im mg-Bereich verarbeitet werden können. Schwankungen in der Produkt- oder Packungsmittelfeuchte werden durch das erfindungsgemäße Verfahren ebenfalls kompensiert.

Die Auswertung der durch das erfindungsgemäße Verfahren erhaltenen Datensätze erfolgt wie nachstehend beschrieben.

Die Massen der Verpackung (Tara) und des Meßgutes (Netto) werden mit $m_T$, $m_N$ bezeichnet. Entweder die Verpackung oder der Inhalt dürfen wasserhaltig sein. Die entweder in der Leerkapsel oder im Füllgut enthaltene Wassermasse wird nachstehend mit $m_{H2O}$ bezeichnet.

Die durch die Mikrowellenmeßmethode bestimmten beiden Meßsignale für Verstimmung und Verbreiterung der Resonanz werden nachstehend mit $F = f(L) - f(0)$ bzw. $B = b(0) - b(L)$ bezeichnet und das Resultat der Bruttoverwiegung mit *C*.

Die Verstimmung des Mikrowellenresonators *F* als auch die Verbreiterung der Resonanz *B* sind proportional zur Masse der jeweils in den Resonator eingebrachten Probe. Mithin können die beiden Mikrowellenmeßsignale in der folgenden Form dargestellt werden

$$F = f_T m_T + f_{H2O} m_{H2O} + f_N m_N, \tag{1}$$

$$B = b_T m_T + b_{H2O} m_{H2O} + b_N m_N . \tag{2}$$

Hierbei stehen die Koeffizienten $f_T$, $f_{H20}$, $f_N$ und $b_T$, $b_{H20}$, $b_N$ für die jeweils substanzspezifischen Proportionalitätskonstanten.

Da die Mikrowellendämpfung in erster Linie vom Wassergehalt des Meßgutes abhängt, wird das Meßsignal $B$ im Wesentlichen durch den Term $b_{H20}m_{H20}$ in Gleichung (2) bestimmt. Schwankungen in entweder der Produkt- oder der Packungsmittelfeuchte werden also durch Berücksichtigung des Meßwertes B kompensiert.

Für das Bruttowiegesignal gilt

$$C = m_T + m_N .  \tag{3}$$

Nach Inversion des linearen Gleichungssystems (1),(2),3) und Auflösung nach der gesuchten Nettoeinwaage erhält man $m_N$ als Linearkombination der erhobenen Meßdaten $F$, $B$ , und $C$ in der Form

$$m_N = \alpha F + \beta B + \gamma C + \delta .  \tag{4}$$

Zur Kalibrierung werden die Koeffizienten $\alpha$, $\beta$, $\gamma$, $\delta$ in Gleichung (4) durch lineare Regression aus einer Meßreihe mit Referenzproben bekannter Einwaage bestimmt. Der Koeffizient $\delta$ trägt hier einer möglichen Verschiebung der Kalibrierung durch andere Einflußfaktoren (z.B .Temperatur) Rechnung.

Da die Mikrowellenverstimmung $F$ stets zur jeweiligen Dielektrizitätskonstanten (DK) der in den Resonator eingebrachten Materialen proportional ist, ist das Funktionsprinzip der vorgeschlagenen Apparatur gewährleistet, wenn die DK's von Packmittel und Inhalt hinreichend verschieden sind.

Unter Kapseln werden im Rahmen der vorliegenden Erfindung geschlossene Kapseln verstanden, die durch einen Gehalt an einem Pulver gekennzeichnet sind.

Das Material, aus welchem diese Kapseln bestehen ist erfindungsgemäß ausgewählt aus der Gruppe bestehend aus Gelatine, Cellulosederivaten, Stärke, Stärkederivaten, Chitosan und synthetischen Kunststoffen. Wird Gelatine als Kapselmaterial verwendet, so kann diese im Gemisch mit anderen Zusätzen ausgewählt aus der Gruppe bestehend aus Polyethylenglycol (PEG), bevorzugt PEG 3350, Glycerol, Sorbitol, Propylenglycol, PEO-PPO-Blockcopolymeren und anderen Polyalkoholen und Polyethern zum Einsatz kommen. Besonders bevorzugt wird im Rahmen der vorliegenden Erfindung Gelatine im Gemisch mit PEG, bevorzugt PEG 3350, verwendet. Besonders bevorzugt enthält eine erfindungsgemäße Gelatine-Kapsel PEG in einem Anteil von 1-10% (Gew-%), bevorzugt 3-8 %. Besonders bevorzugte Gelatine-Kapseln enthalten PEG in einem Anteil von 4-6%, wobei ein PEG-Anteil von etwa 5% erfindungsgemäß höchstbevorzugt ist.

Werden Cellulosederivate als Kapselmaterial verwendet, so ist die Verwendung von Hydroxypropylmethylcellulose, Hydroxypropylcellulose, Methylcellulose, Hydroxymethylcellulose und Hydroxyethylcellulose bevorzugt. Besonders bevorzugt wird in diesem Fall Hydroxypropylmethylcellulose (HPMC), besonders bevorzugt HPMC 2910 als Kapselmaterial eingesetzt. Werden als Kapselmaterial synthetische Kunststoffe eingesetzt, so sind diese erfindungsgemäß bevorzugt ausgewählt aus der Gruppe bestehend aus Polyethylen Polycarbonat, Polyester, Polypropylen und Polyethylenterephthalat. Besonders bevorzugt sind als synthetische Kunststoffmaterialien für die erfindungsgemäßen Inhalationskapseln Polyethylen, Polycarbonat oder Polyethylenterephthalat. Wird Polyethylen als eines der erfindungsgemäß besonders bevorzugten Kapselmaterialien verwendet, gelangt vorzugsweise Polyethylen mit einer Dichte zwischen 900 und 1000 kg/m$^3$, bevorzugt von 940 - 980 kg/m$^3$, besonders bevorzugt von 960 kg/m$^3$ (high-density Polyethylen) zur Anwendung.

Die Herstellung der leeren Kapseln kann nach im Stand der Technik bekannten Vorgehensweisen erfolgen. Beispielsweise seien als mögliche Herstellungsverfahren die im Stand der Technik bekannten Prozesse des Tauchverfahrens, Blasdruckverfahrens, Spritzgußverfahrens, Extrusionsverfahrens und Taufziehverfahrens genannt.

Das erfindungsgemäße Verfahren zur Nettoverwiegung von Kapseln zielt ab auf die Bestimmung der genauen Einwaage bzw. Masse $m_N$ des in den Kapseln enthaltenen Pulvers. Bei diesem Pulver handelt es sich vorzugsweise um ein für die inhalation vorgesehenes Pulver, welches neben einem Wirkstoff einen pharmazeutisch verträglichen Hilfsstoff enthält.

Als pharmazeutisch verträgliche Hilfsstoffe seien beispielsweise genannt Monosaccharide (z.B. Glucose oder Arabinose), Disaccharide (z.B. Lactose, Saccharose, Maltose, Trehalose), Oligo- und Polysaccharide (z.B. Dextrane), Polyalkohole (z.B. Sorbit, Mannit, Xylit), Salze (z.B. Natriumchlorid, Calciumcarbonat) oder Mischungen dieser Hilfsstoffe miteinander. Bevorzugt gelangen Mono- oder Disaccharide zur Anwendung, wobei die Verwendung von Lactose oder Glucose, insbesondere, aber nicht ausschließlich in Form ihrer Hydrate, bevorzugt ist. Als besonders bevorzugt im Sinne der Erfindung gelangt Lactose, höchst bevorzugt Lactosemonohydrat als Hilfsstoff zur Anwendung.

Als Wirkstoffe werden im Rahmen der vorliegenden Erfindung Verbindungen verstanden, die bevorzugt ausgewählt sind aus der Gruppe der Anticholinergika, Betamimetica, Dopaminagonisten, Antiallergika, Leukotrien-Antagonisten und Corticosteroiden, sowie gegebenenfalls Wirkstoffkombinationen davon.

Als Anticholinergika kommen bevorzugt Verbindungen ausgewählt aus der Gruppe der Tiotropiumsalze, Ipratropiumsalze, Oxitropiumsalze,

Salze der aus der WO 02/32899 bekannten Verbindungen

N-Methyl-2,2-Diphenylpropionsäuretropenolester,

N-Methyl-2,2-Diphenylpropionsäurescopinester,

N-Methyl-2-Fluor-2,2-Diphenylessigsäurescopinester und

N-Methyl-2-Fluor-2,2-Diphenylessigsäuretropenolester

sowie Salze der aus der WO 02/32899 bekannten Verbindungen

N-Methyl-3,3',4,4'-Tetrafluorbenzilsäuretropenolester,

N-Methyl-3,3',4,4'-Tetrafluorbenzilsäurescopinester;

N-Methyl-4,4'-Dichlorbenzilsäurescopinester,

N-Methyl- 4,4'-Difluorbenzilsäurescopinester,

N-Methyl-3,3'-Difluorbenzilsäuretropenolester,

N-Methyl- 3,3'-Difluorbenzilsäurescopinester und

N-Ethyl-4,4'-Difluorbenzilsäuretropenolester gegebenenfalls in Form ihrer Hydrate und Solvate, in Betracht.

Unter Salzen sind dabei diejenigen Verbindungen zu verstehen, die neben den vorstehend genannten Kationen als Gegenion ein einfach negativ geladenes Anion ausgewählt aus der Gruppe Chlorid, Bromid und Methansulfonat enthalten.

Besonders bevorzugt kommen als Wirkstoffe im Rahmen der vorliegenden Erfindung die Bromide oder Methansulfonate der vorstehend genannten Strukturen in Betracht.

Von herausragendem Interesse sind im Rahmen der vorliegenden Erfindung beispielsweise Anticholinergika Tiotropiumbromid, Ipratropiumbromid, Oxitropiumbromid, 2,2-Diphenylpropionsäuretropenolester-methobromid, 2,2-Diphenylpropionsäurescopinester-methobromid, 2-Fluor-2,2-Diphenylessigsäurescopinester-methobromid, 2-Fluor-2,2-Diphenylessigsäuretropenolester-methobromid, 3,3',4,4'-Tetrafluorbenzilsäuretropenolester-methobromid, 3,3',4,4'-Tetrafluorbenzilsäurescopinester-methobromid; 4,4'-Dichlorbenzilsäurescopinestermethobromid, 4,4'-Difluorbenzilsäurescopinester-methobromid, 3,3'-Difluorbenzilsäuretropenolester-methobromid, 3,3'-Difluorbenzilsäurescopinester-methobromid und 4,4'-Difluorbenzilsäuretropenolester-ethylbromid woebi der Tiotropiumbromid, dem Ipratropiumbromid, dem 2,2-Diphenylpropionsäuretropenolester-methobromid, dem 2,2-Diphenylpropionsäurescopinester-methobromid, dem 2-Fluor-2,2-Diphenylessigsäurescopinester-methobromid sowie dem 2-Fluor-2,2-Diphenylessigsäuretropenolester-methobromid besondere Bedeutung zukommen.

Von herausragender Bedeutung ist hierbei das Tiotropiumbromid, besonders bevorzugt in Form seines aus der WO 02/30928 bekannten kristallinen Monohydrats.

Als Betamimetika kommen erfindungsgemäß bevorzugt diejenigen Verbindungen in Betracht, die ausgewählt sind aus der Gruppe bestehend aus Bambuterol, Bitolterol, Carbuterol, Clenbuterol, Fenoterol, Formoterol, Hexoprenalin, Ibuterol, Pirbuterol, Procaterol, Reproterol, Salbutamol, Salmeterol, Sulfonterol, Terbutalin, Tolubuterol, 4-Hydroxy-7-[2-{[2-{[3-(2-phenylethoxy)propyl]sulfonyl}ethyl]-amino}ethyl]-2(3H)-benzothiazolon, 1-(2-Fluoro-4-hydroxyphenyl)-2-[4-(1-benzimidazolyl)-2-methyl-2-butylamino]ethanol, 1-[3-(4-Methoxybenzyl-amino)-4-hydroxyphenyl]-2-[4-(1-benzimidazolyl)-2-methyl-2-butylamino]ethanol, 1-[2H-5-Hydroxy-3-oxo-4H-1,4-benzoxazin-8-yl]-2-[3-(4-N,N-dimethylaminophenyl)-2-methyl-2-propylamino]ethanol, 1-[2H-5-Hydroxy-3-oxo-4H-1,4-benzoxazin-8-yl]-2-[3-(4-methoxyphenyl)-2-methyl-2-propylamino]ethanol, 1-[2H-5-Hydroxy-3-oxo-4H-1,4-benzoxazin-8-yl]-2-[3-(4-n-butyloxyphenyl)-2-methyl-2-propylamino]ethanol, 1-[2H-5-Hydroxy-3-oxo-4H-1,4-benzoxazin-8-yl]-2-{4-[3-(4-methoxyphenyl)-1,2,4-triazol-3-yl]-2-methyl-2-butylamino}ethanol, 5-Hydroxy-8-(1-hydroxy-2-isopropylaminobutyl)-2H-1,4-benzoxazin-3-(4H)-on, 1-(4-Amino-3-chloro-5-trifluormethylphenyl)-2-tert.-butylamino)ethanol und 1-(4-Ethoxycarbonylamino-3-cyano-5-fluorophenyl)-2-(tert.-butylamino)ethanol, gegebenenfalls in Form ihrer Racemate, ihrer Enantiomere, ihrer Diastereomere, sowie gegebenenfalls ihrer pharmakologisch unbedenklichen Säureadditionssalze und Hydrate. Besonders bevorzugt gelangen als Betamimetika solche Wirkstoffe zur Anwendung, die ausgewählt sind aus der Gruppe bestehend aus Fenoterol, Formoterol, Salmeterol, Salbutamol, 1-[3-(4-Methoxybenzyl-amino)-4-hydroxyphenyl]-2-[4-(1-benzimidazolyl)-2-methyl-2-butylamino]ethanol, 1-[2H-5-Hydroxy-3-oxo-4H-1,4-benzoxazin-8-yl]-2-[3-(4-N,N-dimethylaminophenyl)-2-methyl-2-propylamino]ethanol, 1-[2H-5-Hydroxy-3-oxo-4H-1,4-benzoxazin-8-yl]-2-[3-(4-methoxyphenyl)-2-methyl-2-propylamino]ethanol, 1-[2H-5-Hydroxy-3-oxo-4H-1,4-benzoxazin-8-yl]-2-[3-(4-n-butyloxyphenyl)-2-methyl-2-propylamino]ethanol, 1-[2H-5-Hydroxy-3-oxo-4H-1,4-benzoxazin-8-yl]-2-{4-[3-(4-methoxyphenyl)-1,2,4-triazol-3-yl]-2-methyl-2-butylamino}ethanol, gegebenenfalls in Form ihrer Racemate, ihrer Enantiomere, ihrer Diastereomere, sowie gegebenenfalls ihrer pharmakologisch unbedenklichen Säureadditionssalze und Hydrate. Von den vorstehend genannten Betamimetika kommt hierbei den Verbindungen Formoterol und Salmeterol gegebenenfalls in Form ihrer Racemate, ihrer Enantiomere, ihrer Diastereomere, sowie gegebenenfalls ihrer pharmakologisch unbedenklichen Säureadditionssalze und Hydrate besondere Bedeutung zu.

Beispeilsweise sind die Säureadditionssalze der Betamimetika ausgewählt aus der Gruppe bestehend aus Hydrochlorid, Hydrobromid, Sulfat, Phosphat, Fumarat Methansulfonat und Xinafoat.

Besonders bevorzugt sind die Salze im Falle des Formoterols ausgewählt aus Hydrochlorid, Sulfat und Fumarat, von denen das Hydrochlorid und Fumarat besonders bevorzugt sind. Erfindungsgemäß von herausragender Bedeutung ist Formoterolfumarat. Besonders bevorzugt sind die Salze im Falle des Salmeterols ausgewählt aus Hydrochlorid, Sulfat und Xinafoat, von denen das Xinafoat besonders bevorzugt ist.

Im Rahmen der vorliegenden Erfindung werden unter Corticosteroiden, die erfindungsgemäß zum Einsatz gelangen können, Verbindungen verstanden, die ausgewählt sind aus der Gruppe bestehend aus Flunisolide, Beclomethasone, Triamcinolone, Budesonid, Fluticasone, Mometasone, Ciclesonide, Rofleponide, GW 215864, KSR 592, ST-126 und Dexametasone. Bevorzugt sind im Rahmen der vorliegenden Erfindung die Corticosteroide ausgewählt aus der Gruppe bestehend aus Flunisolide, Beclomethasone, Triamcinolone, Budesonid, Fluticasone, Mometasone, Ciclesonide und Dexametasone, wobei hier dem Budesonid, Fluticasone, Mometasone und Ciclesonide, insbesondere dem Budesonid und dem Fluticason eine besondere Bedeutung zukommt. Gegebenfalls wird im Rahmen der vorliegenden Patentanmeldung statt der Bezeichnung Corticosteroide auch nur die Bezeichnung Steroide verwendet. Eine Bezugnahme auf Steroide schließt im Rahmen der vorliegenden Erfindung eine Bezugnahme auf Salze oder Derivate, die von den Steroiden gebildet werden können, mit ein. Als mögliche Salze oder Derivate werden beispielsweise genannt: Natriumsalze, Sulfobenzoate, Phosphate, Isonicotinate, Acetate, Propionate, Dihydrogenphosphate, Palmitate, Pivalate oder Furoate. Gegebenenfalls können die Corticosteroide auch in Form ihrer Hydrate vorliegen.

Im Rahmen der vorliegenden Erfindung werden unter Dopamin-Agonisten Verbindungen verstanden, die ausgewählt sind aus der Gruppe bestehend aus Bromocriptin, Cabergolin, Alpha-Dihydroergocryptin, Lisurid, Pergolid, Pramipexol, Roxindol, Ropinirol, Talipexol, Tergurid und Viozan. Bevorzugt werden im Rahmen der vorliegenden Erfindung Dopamin-Agonisten eingesetzt, die ausgewählt sind aus der Gruppe bestehend aus Pramipexol, Talipexol und Viozan, wobei Pramipexol eine besondere Bedeutung zukommt. Eine Bezugnahme auf die vorstehend genannten Dopamin-Agonisten schließt im Rahmen der vorliegenden Erfindung eine Bezugnahme auf deren gegebenenfalls existierende pharmakologisch verträgliche Säureadditionssalze und gegebenenfalls deren Hydrate ein. Unter den physiologisch verträglichen Säureadditionssalzen, die von den vorstehend genannten Dopaminagonisten gebildet werden können, werden beispielsweise pharmazeutisch verträgliche Salze verstanden, die ausgewählt aus den Salzen der Salzsäure, Bromwasserstoffsäure, Schwefelsäure, Phosphorsäure, Methansulfonsäure, Essigsäure, Fumarsäure, Bernsteinsäure, Milchsäure, Zitronensäure, Weinsäure und Maleinsäure sind.

Als Beispiel für Antiallergika, die erfindungsgemäß zum Einsatz kommen können, seien genannt Epinastin, Cetirizin, Azelastin, Fexofenadin, Levocabastin, Loratadin, Mizolastin, Ketotifen, Emedastin, Dimetinden, Clemastin, Bamipin, Cexchlorpheniramin, Pheniramin, Doxylamin, Chlorphenoxamin, Dimenhydrinat, Diphenhydramin, Promethazin, Ebastin, Desloratidin und Meclozin. Bevorzugte Antiallergika, die im Rahmen der vorliegenden Erfindung zum Einsatz gelangen können, sind ausgewählt aus der Gruppe bestehend aus Epinastin, Cetirizin, Azelastin, Fexofenadin, Levocabastin, Loratadin, Ebastin, Desloratidin und Mizolastin wobei Epinastin und Desloratidin besonders bevorzugt sind. Eine Bezugnahme auf die vorstehend genannten Antiallergika schließt im Rahmen der vorliegenden Erfindung eine Bezugnahme auf deren gegebenenfalls existierende pharmakologisch verträgliche Säureadditionssalze ein.

Die nachfolgenden Ausführungsbeispiele dienen der weitergehenden Erläuterung der Erfindung. Die folgenden Meßresultate wurden an einem Ensemble von 25 mit einem Tiotropiumbromid-haltigen Inhalationspulver befüllten Kapseln erzielt.

Die Mikrowellenmeßwerte $F$ und $B$ wurden mit Hilfe einer durch die Firma TEWS Elektronik (Sperberhorst 10, D-22459 Hamburg) konstruierten und gelieferten Mikrowellen-Feuchtemeßanlage MW3010, der Mikrowellenauswerteelektronik MW3010 und dem Resonator E83/8 gemessen. Die Bruttoverwiegungen zur Ermittlung des Meßwertes C wurden mit Hilfe einer Analysenwaage AX105 der Firma Mettler Toledo GmbH (Im Langacher, CH-8606 Greifensee, Schweiz) bei der reduzierten Ablesegenauigkeit von 0.1mg vorgenommen.

Zur Kalibrierung der Apparatur und zur Bewertung der erzielbaren Genauigkeit des Verfahrens war es notwenig, die wahren Einwaagen $m_N$ der 25 Kapseln genau zu kennen. Dazu wurden die Kapseln manuell durch einen Handpulverstechheber befüllt. Durch jeweilige Verwiegung der einzelnen Kapseln vor und nach der Befüllung auf der o.g. Mettler Analysewaage bei einer Ablesegenauigkeit von 0.01mg wurde das Nettofüllgewicht ermittelt.

Tabelle 1 faßt alle Meß- und Einwaagewerte zusammen. Die ersten 15 Proben wurden zur Gerätekalibrierung verwendet. Zu diesem Zweck wurden die Koeffizienten $\alpha, \beta, \gamma, \delta$ aus Gleichung(4) durch lineare Regression der Meßwerte F, B, C mit den bekannten Einwaagewerten $m_N$ berechnet.

Als Zusammengang zwischen Meßsignal und Einwaage wurde daraus erhalten

$$m_N = -0.18105 \frac{mg}{MHz} * A + 0.0438 \frac{mg}{MHz} * B + 1.470 * C . \qquad (5)$$

Als Standardabweichung zwischen den 15 Kalibrierdaten und den nach Kalibrierung errechneten Vorhersagen (Kreuzkalibrierung) erhält man $\sigma_{Kalibrierdaten}$ = 0.33 *mg*.

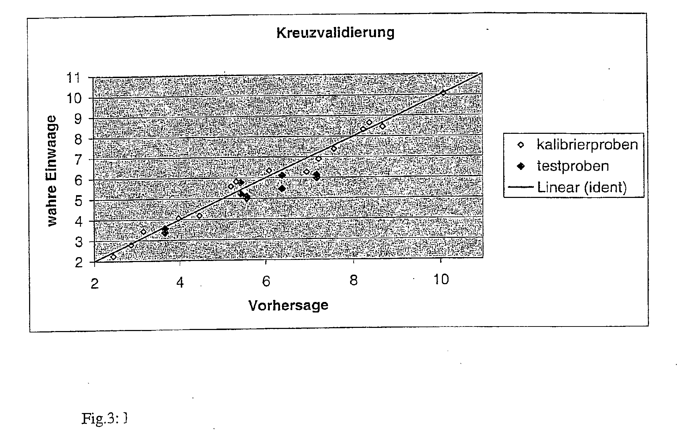

Nach der so durchgeführten Kalibrierung wurden die restlichen 10 Kapseln zum Test der Genauigkeit der vorgeschlagenen Verwiegemethode herangezogen. Hier liefert der Vergleich zwischen den wahren Einwaagewerten und den auf der Kalibrierung (5) basierenden Vorhersagen eine Standardabweichung von $\sigma_{Testproben}$ = 0.68 *mg*. Fig. 3 faßt alle Kalibrier- und Testdaten im Kreuzvalidierungsdiagramm zusammen.

Tab.1: Meßwerte der 25 Kalibrier und Testproben

| | Probe | Netto [mg] Analyse waage | Brutto [mg] Analyse waage | Tara [mg] Analyse waage | Mikrowelle F [MHz] | Mikrowelle B [MHz] | Brutto [mg] C [mg] | Vorhesage $m_N$ [mg] nach Gl. (5) |
|---|---|---|---|---|---|---|---|---|
| Kalibrierproben | 1 | 2.44 | 50.23 | 47.79 | 418.8594 | 103.9680936 | 50 | 2.24 |
| | 2 | 2.85 | 51.06 | 48.21 | 424.9063 | 104.6123785 | 51.1 | 2.79 |
| | 3 | 3.14 | 50.31 | 47.17 | 413.8125 | 100.6091802 | 50.3 | 3.45 |
| | 4 | 3.97 | 52.04 | 48.07 | 425.9063 | 104.3166723 | 52.1 | 4.07 |
| | 5 | 4.46 | 52.29 | 47.83 | 424.8594 | 102.5303456 | 52.1 | 4.18 |
| | 6 | 5.19 | 52.46 | 47.27 | 416.9063 | 98.36373737 | 52.2 | 5.58 |
| | 7 | 5.31 | 53.51 | 48.2 | 429.9063 | 103.8392956 | 53.8 | 5.82 |
| | 8 | 6.08 | 54.36 | 48.28 | 430.875 | 102.979476 | 54.3 | 6.34 |
| | 9 | 6.94 | 53.2 | 46.26 | 421.9375 | 101.2003059 | 53.2 | 6.27 |
| | 10 | 7.56 | 54.7 | 47.14 | 427.9375 | 101.101946 | 54.7 | 7.38 |
| | 11 | 8.23 | 56.18 | 47.95 | 434.9531 | 101.3368326 | 56.2 | 8.33 |
| | 12 | 7.21 | 55.41 | 48.2 | 435.9453 | 102.809727 | 55.3 | 6.89 |
| | 13 | 8.37 | 56.63 | 48.26 | 434.9375 | 101.9294894 | 56.4 | 8.65 |
| | 14 | 8.67 | 56.97 | 48.3 | 440 | 101.7243854 | 56.9 | 8.46 |
| | 15 | 10.08 | 58.3 | 48.22 | 440.9297 | 101.7071026 | 58.1 | 10.06 |
| Testprobe | 16 | 7.16 | 56.27 | 49.11 | 451.0703 | 111.1018252 | 56.3 | 5.98 |
| | 17 | 7.16 | 56.27 | 49.11 | 451.1563 | 111.1708615 | 56.4 | 6.12 |
| | 18 | 5.56 | 53.51 | 47.95 | 432.0078 | 107.2317522 | 53.4 | 5.00 |

| 19 | 5.56 | 53.51 | 47.95 | 434.0625 | 107.6035326 | 53.7 | 5.09 |
|---|---|---|---|---|---|---|---|
| 20 | 6.38 | 54.16 | 47.78 | 433.0313 | 106.4290683 | 54.3 | 6.10 |
| 21 | 6.38 | 54.16 | 47.78 | 435.0625 | 106.8360262 | 54.1 | 5.46 |
| 22 | 5.42 | 52.74 | 47.32 | 424.0703 | 103.103133 | 52.7 | 5.23 |
| 23 | 5.42 | 52.74 | 47.32 | 426.125 | 103.9186604 | 53.3 | 5.77 |
| 24 | 3.65 | 51.65 | 48 | 426.0156 | 105.6539372 | 51.6 | 3.37 |
| 25 | 3.65 | 51.65 | 48 | 424.0938 | 105.4024385 | 51.5 | 3.56 |

## Patentansprüche

1. Verfahren zur zerstörungsfreien Nettoverwiegung von Kapseln, bei dem in einem ersten Verfahrensschritt durch einen Prozessor gesteuert in einem Mikrowellen-Generator eine elektromagnetische Strahlung variabler Frequenz erzeugt und einem als Resonator ausgebildeten Probenapplikator zugeführt wird, bei dem das aus dem Applikator austretende Mikrowellensignal einer Detektor-Diode zugeführt wird, aus deren Signalen über einen Analog/Digital-Wandler vom Rechner als primäre Meßgrößen b(0) und f(0) ermittelt werden, wobei b(0) die Halbwertsbreite bei der Resonanzfrequenz f(0) des mit einer Messprobe in Wirkverbindung stehenden Applikators ist, **dadurch gekennzeichnet, dass** mit dem Resonator eine Materialprobe so in Verbindung gebracht wird, dass das elektrische Feld des Resonators beim Übergang in das Material der Probe allgemein parallel zur Oberfläche verläuft und dass aus den primären Messgrössen b(0) und f(0) die Messsignale

$$F = f(L) - f(0) \ \text{bzw.} \ B = b(0) - b(L)$$

erhalten werden, wobei F und B für die Mikrowellenmesssignale der Verstimmung und Verbreiterung der Resonanz stehen, mit $f(L)$ und $b(L)$ gleich konstante materialabhängige Bezugsgrößen,
und bei dem in einem zweiten Schritt eine gravimetrische Bruttoverwiegung zur Bestimmung des Bruttowiegesignals

$$C = m_T + m_N,$$

in dem die Grösse $m_T$ für die Masse des leeren Gebindes und die Grösse $m_N$ für die zu bestimmende Nettomasse steht, durchgeführt wird, und abschließend aus den erhaltenen Messsignalen $F$, $B$ und $C$ die zu bestimmende Nettomasse $m_N$ gemäß nachfolgender Relation

$$m_N = \alpha F + \beta B + \gamma C + \delta$$

rechnerisch bestimmt wird, wobei die Koeffizienten $\alpha$ $\beta$, $\gamma$ und $\delta$ zuvor über Kalibrierung des Messsystems durch lineare Regression aus einer Meßreihe mit Referenzproben bekannter Einwaage bestimmt wurden.

2. Verfahren nach Anspruch 1, **dadurch gekennzeichnet, dass** befüllte Kapseln verwogen werden, deren Material ausgewählt ist aus der Gruppe bestehend aus Gelatine, Cellulosederivaten, Stärke, Stärkederivaten, Chitosan und synthetischen Kunststoffen.

3. Verfahren nach Anspruch 1 oder 2, **dadurch gekennzeichnet, dass** die Kapseln ein Pulver enthalten, welches einen Wirkstoff und ggebenenfalls einen pharmazeutisch verträglichen Hilfsstoff enthält.

**4.** Verfahren nach Anspruch 3, **dadurch gekennzeichnet, dass** der Wirkstoff ausgewählt ist aus der Gruppe der Anticholinergika, Betamimetica, Dopaminagonisten, Antiallergika, Leukotrien-Antagonisten und Corticosteroiden, sowie gegebenenfalls Wirkstoffkombinationen davon.

**5.** Verfahren nach einem der Ansprüche 1 bis 4, **dadurch gekennzeichnet, dass** die Meßsignalwerte F und B mittels einer Vorrichtung bestimmt werden, welche **gekennzeichnet ist durch** einen von einem Prozessor (2) digital abstimmbaren Mikrowellengenerator (3) mit variabler Frequenz, der mit einer Ankopplungssonde (5) verbunden ist, die in einem Applikator (4) zur Messung der Meßsignale $F$ und $B$ einer Probe (11) angeordnet ist, der eine weitere Ankopplungssonde (6) aufweist, die über einen Mikrowellen-Verstärker bzw. -Abschwächer (7) mit einer Detektor-Diode (8) verbunden ist, deren Signalausgang mit dem Prozessor (2) verbunden ist.

**6.** Verfahren nach einem der Ansprüche 1 bis 5, **dadurch gekennzeichnet, dass** zur Bestimmung des Bruttowiegesignals C eine gravimetrische Wiegezelle zum Einsatz gelangt, die das Gewicht mit einer Standardabweichung von +/- 0.1 mg bestimmt.

**7.** Verfahren nach einem der Ansprüche 1 bis 6, **dadurch gekennzeichnet, dass** es mittels einer Vorrichtung durchgeführt wird, welche einen Trichter (1') aufweist, aus dem die befüllten und verschlossenen Kapseln mittels einer Vereinzelungseinheit (2') seriell und/oder einzeln durch den Mikrowellenresonator (3') geführt werden, welche ferner eine sich daran anschließende Verteilereinheit (4') aufweist, durch welche der Transport der Kapseln parallelisiert wird und durch welche die Kapseln auf mehrere Spuren verteilt werden, in denen sich die gravimetrischen Wiegezellen (5') befinden, welche ferner eine computergestützte Datenerfassungseinheit (7') aufweist, die mit den Einheiten (3'), (4') und (5') verbunden ist und für die richtige Zuordnung der Meßwerte zu den einzelnen Kapseln sorgt und die Auswertung durchführt und welche schließlich eine Weiche (6') aufweist, durch die Kapseln mit fehlgewichtiger Einwaage ausgeschieden werden.

**8.** Vorrichtung zur Ausführung des Verfahrens nach Anspruch 1, welche einen Trichter (1') aufweist, aus dem die befüllten und verschlossenen Kapseln mittels einer Vereinzelungseinheit (2') seriell und/oder einzeln durch den Mikrowellenresonator (3') geführt werden, welche ferner eine sich daran anschließende Verteilereinheit (4') aufweist, durch welche der Transport der Kapseln parallelisiert wird und durch welche die Kapseln auf mehrere Spuren verteilt werden, in denen sich die gravimetrischen Wiegezellen (5') befinden, welche ferner eine computergestützte Datenerfassungseinheit (7') aufweist, die mit den Einheiten (3'), (4') und (5') verbunden ist und für die richtige Zuordnung der Meßwerte zu den einzelnen Kapseln sorgt und die Auswertung durchführt und welche schließlich eine Weiche (6') aufweist, durch die Kapseln mit fehlgewichtiger Einwaage ausgeschieden werden.

**Claims**

**1.** Process for the non-destructive net weighing of capsules, wherein in a first step of the process variable frequency electromagnetic radiation is generated in a microwave generator under the control of a processor and is supplied to a probe applicator in the form of a resonator, the microwave signal leaving the applicator being supplied to a detector diode from whose signals b(0) and f(0) are determined by the computer as primary measurement values by means of an analogue-to-digital converter, b(0) being the half-power width at the resonant frequency f(0) of the applicator which is operatively connected to a measuring probe, **characterised in that** a sample of material is connected to the resonator such that the electric field of the resonator runs generally parallel to the surface on passing into the material of the probe and **in that**, from the primary measurement values b(0) and f(0), the measuring signals

$$F = f(L) - f(0) \quad or \quad B = b(0) - b(L)$$

are obtained, where F and B denote the microwave measuring signals of the detuning and propagation of the resonance, with $f(L)$ and $b(L)$ being equal to constant material-dependent reference values, and wherein in a second step gravimetric gross weighing is carried out in order to determine the gross weight signal

$$C = m_T + m_N,$$

wherein the magnitude $m_T$ represents the mass of the empty packaging and the magnitude $m_N$ represents the net mass to be determined, and finally from the measuring signals *F, B* and *C* obtained the net mass $m_N$ to be determined is computed according to the following equation

$$m_N = \alpha F + \beta B + \gamma C + \delta$$

the coefficients $\alpha$ $\beta$, $\gamma$ and $\delta$ having been determined beforehand by calibration of the measuring system by linear regression from a measuring series with reference samples of known net weight.

2. Process according to claim 1, **characterised in that** filled capsules are weighed, the material of which is selected from the group consisting of gelatine, cellulose derivatives, starch, starch derivatives, chitosan and synthetic plastics.

3. Process according to claim 1 or 2, **characterised in that** the capsules hold a powder which contains an active substance and optionally a pharmaceutically acceptable excipient.

4. Process according to claim 3, **characterised in that** the active substance is selected from the group comprising the anticholinergics, betamimetics, dopamine agonists, antiallergics, leukotriene antagonists and corticosteroids and optionally combinations of active substances thereof.

5. Process according to one of claims 1 to 4, **characterised in that** the measured signal values F and *B* are determined by means of an apparatus which is **characterised by** a variable-frequency microwave generator (3) which can be digitally tuned by a processor (2), this generator being connected to a coupling probe (5) which is disposed in an applicator (4) for measuring the measuring signals F and B of a sample (11), said applicator (4) having another coupling probe (6) which is connected via a microwave amplifier or attenuator (7) to a detector diode (8) the signal output of which is connected to the processor (2).

6. Process according to one of claims 1 to 5, **characterised in that** to determine the gross weight signal C a gravimetric weighing cell is used which determines the weight with a standard deviation of $\pm$ 0.1 mg.

7. Process according to one of claims 1 to 6, **characterised in that** it is carried out using an apparatus which comprises a hopper (1') from which the filled and sealed capsules are guided serially and/or individually by means of a separating unit (2') through the microwave resonator (3'), which also comprises an adjacent distributor unit (4') by means of which the capsules are arranged parallel for transporting and by means of which the capsules are distributed onto several pathways where the gravimetric weighing cells (5') are located, and which also comprises a computer-aided data collecting unit (7') which is connected to the units (3'), (4') and (5') and ensures that the measurements are correctly assigned to the individual capsules and performs the evaluation and which finally has a reject channel (6') through which capsules of the wrong weight are rejected.

8. Apparatus for carrying out the process according to claim 1, which comprises a hopper (1') from which the filled and sealed capsules are guided serially and/or individually by means of a separating unit (2') through the microwave resonator (3'), which also comprises an adjacent distributor unit (4') by means of which the capsules are arranged parallel for transporting and by means of which the capsules are distributed onto several pathways where the gravimetric weighing cells (5') are located, and which also comprises a computer-aided data collecting unit (7') which is connected to the units (3'), (4') and (5') and ensures that the measurements are correctly assigned to the individual capsules and performs the evaluation and which finally has a reject channel (6') through which capsules of the wrong weight are rejected.

**Revendications**

1. Procédé de détermination non destructive du poids net de capsules, dans lequel, dans une première étape du procédé, est produit un rayonnement électromagnétique de fréquence variable contrôlé par un processeur dans un générateur de micro-ondes, qui est acheminé à un applicateur d'échantillons développé comme résonateur, dans lequel le signal micro-ondes émis depuis l'applicateur est acheminé à une diode détectrice à partir des signaux de laquelle sont calculés b(0) et f(0) comme grandeurs de mesure primaires par le calculateur via un convertisseur analogique/numérique, où b(0) est la largeur de valeur moyenne à la fréquence de résonance f(0) de l'applicateur en liaison avec une sonde de mesure, procédé **caractérisé en ce qu'**un échantillon de matériau est raccordé au résonateur de telle sorte que le champ électrique du résonateur s'étend généralement parallèlement à la surface quand il passe dans le matériau de l'échantillon et **en ce qu'**on obtient à partir des grandeurs de mesure primaires $b(0)$ et $f(0)$ les signaux de mesure

$$F = f(L) - f(0), \text{ et resp. } B = b(0) - b(L)$$

où $F$ et $B$ représentent les signaux micro-ondes du désaccord de fréquence et de l'élargissement de fréquence à la résonance, avec $f(L)$ et $b(L)$ grandeurs de référence constantes fonctions du matériau,
et dans lequel, dans une deuxième étape est effectuée une détermination gravimétrique de poids brut pour déterminer le signal de poids brut

$$C = m_T + m_N$$

où la grandeur $m_T$ représente la masse de l'emballage vide et la grandeur $m_N$ représente la masse nette que l'on détermine, et où finalement est déterminée par calcul la masse nette $m_N$ à partir des signaux de mesure obtenus $F$, $B$ et $C$ selon la relation suivante :

$$m_N = \alpha F + \beta B + \gamma C + \delta$$

dans laquelle les coefficients $\alpha$, $\beta$, $\gamma$ et $\delta$ ont été déterminés préalablement par étalonnage du système de mesure par régression linéaire à partir d'une série de mesures avec des échantillons de référence de poids connus.

2. Procédé selon la revendication 1, **caractérisé en ce qu'**on pèse des capsules remplies dont le matériau est choisi dans le groupe constitué de la gélatine, de dérivés de la cellulose, de l'amidon, de dérivés de l'amidon, du chitosan et de matières plastiques synthétiques.

3. Procédé selon la revendication 1 ou 2, **caractérisé en ce que** les capsules contiennent une poudre qui contient une substance active et le cas échéant un adjuvant pharmaceutiquement compatible.

4. Procédé selon la revendication 3, **caractérisé en ce que** la substance active est choisie dans le groupe des anticholinergiques, des bétamimétiques, des agonistes de la dopamine, des antiallergiques, des antagonistes de la leucotrine et des corticostéroïdes, ainsi que leurs combinaisons le cas échéant.

5. Procédé selon une des revendications 1 à 4, **caractérisé en ce que** les valeurs des signaux de mesure $F$ et $B$ sont déterminées au moyen d'un appareil qui est **caractérisé par** un générateur de micro-ondes (3) de fréquence variable réglable numériquement par un processeur (2), le générateur étant relié à une sonde de couplage (5) qui est disposée dans un applicateur (4) pour la mesure des signaux de mesure $F$ et $B$ d'un échantillon (11), qui présente une autre sonde de couplage (6) qui est liée à une diode détectrice (8) via un intensificateur ou un réducteur de micro-ondes (7), dont la sortie de signal est liée au processeur (2).

6. Procédé selon une des revendications 1 à 5, **caractérisé en ce que** pour déterminer le signal de poids brut $C$, on a recours à une cellule de pesée gravimétrique qui détermine le poids avec un écart type de $\pm$ 0,1 mg.

7. Procédé selon une des revendications 1 à 6, **caractérisé en ce qu'**il est effectué au moyen d'un appareil qui présente un entonnoir (1') d'où les capsules remplies et scellées sont acheminées au moyen d'une unité de séparation (2') les unes à la suite des autres et/ou une par une au travers du résonateur à micro-ondes (3'), lequel présente en outre une unité de répartition (4') qui s'y rattache, par laquelle le transport des capsules est mis en parallèle et par laquelle les capsules sont réparties sur plusieurs pistes où se trouvent les cellules de pesée gravimétrique (5'), qui présente en outre une unité de saisie de données assistée par ordinateur (7') qui est liée aux unités (3'), (4') et (5'), et qui veille à la bonne affectation des valeurs de mesure à chaque capsule et qui réalise l'évaluation, et qui finalement présente un dispositif d'aiguillage (6') grâce auquel les capsules au poids non conforme sont séparées des autres.

**8.** Dispositif de réalisation du procédé selon la revendication 1, qui présente un entonnoir (1') d'où les capsules remplies et scellées sont acheminées au moyen d'une unité de séparation (2') les unes à la suite des autres et/ou une par une au travers du résonateur à micro-ondes (3'), lequel présente en outre une unité de répartition (4') qui s'y rattache, par laquelle le transport des capsules est mis en parallèle et par laquelle les capsules sont réparties sur plusieurs pistes où se trouvent les cellules de pesée gravimétrique (5'), qui présente en outre une unité de saisie de données assistée par ordinateur (7') qui est liée aux unités (3'), (4') et (5'), et qui veille à la bonne affectation des valeurs de mesure à chaque capsule et qui réalise l'évaluation, et qui finalement présente un dispositif d'aiguillage (6') grâce auquel les capsules au poids non conforme sont séparées des autres.

Fig 1

Fig 2:

Fig.3: }